# EUROPEAN PATENT APPLICATION

(11) **EP 2 489 435 A2**
(43) Date of publication of application: **22.08.2012**
(21) Application number: 12155968.6
(22) Date of filing: 17.02.2012
(51) Int. Cl.: B01L 3/00, C12M 1/00

(54) **Cell incubator and incubation condition monitoring system**

(30) Priority: 18.02.2011 JP 2011033372
(71) Applicant: Nihon Kohden Corporation, Shinjuku-ku Tokyo (JP); Tokyo Women's Medical University, Tokyo 162-8666 (JP)
(72) Inventor: Oura, Mitsuhiro, Shinjuku-ku, Tokyo (JP); Okano, Teruo, Shinjuku-du, Tokyo 162-8666 (JP); Shimizu, Tatsuya, Shinjuku-du, Tokyo 162-8666 (JP); Kubo, Hirotsugu, Shinjuku-ku, Tokyo (JP); Suzuki, Katsuyoshi, Shinjuku-ku, Tokyo (JP); Takeda, Sunao, Shinjuku-ku, Tokyo (JP)
(74) Representative: Grünecker, Kinkeldey, Stockmair & Schwanhäusser

(57) **Abstract**

A cell incubator includes: a culture cell accommodating section which accommodates cells to be cultured and a culture solution; and an RFID tag which is attached to the culture cell accommodating section and which is configured to transmit predetermined information to a communicating apparatus in response to reception of a signal that is wirelessly transmitted from the communicating apparatus.

## Description

### BACKGROUND OF THE INVENTION

The present invention relates to a cell incubator and an incubation condition monitoring system, and more particularly to a cell incubator and incubation condition monitoring system which can wirelessly transmit and receive information such as incubation conditions to and from a predetermined communicating apparatus.

For example, a cell culture container which is used in culture of cells such as an artificially fertilized cell has been known (for example, see JP-A-2006-6261). Such a cell culture container is stored in an incubator in which, for example, the temperature and the concentration of carbon dioxide are controlled.

In the case where a plurality of cell culture containers are to be stored in an incubator, information such as the kind of cells in each of the containers and the history of passage is often written on a label pasted to the container. When the information is to be checked, it is required to seek out the desired container in the incubator, and then take out it from the incubator. Although the temperature of the interior of the incubator is controlled, it is impossible to know the temperature deviations of the respective cell culture containers, and temperature history of each of the cell culture containers.

A system for monitoring the conditions during the cell culture has been proposed (for example, see JP-T-2009-533053) . In the system disclosed in JP-T-2009-533053, however, a measurement is performed in a cuvette which is different from a culture container that stores cells to be cultured and a culture medium, and therefore the conditions of cells under culture cannot be correctly known. To comply with this, a method in which a measurement is performed directly on a culture container may be contemplated. In this method, however, there is a fear that a contamination problem arises in cell culture. By contrast, in the case where a cuvette which is different from a culture container is set as the measurement object as in the above-described system, the possibility of contamination is reduced, but, even in the case where contamination occurs in the culture container, it is impossible to detect the contamination when the contamination does not occur in the cuvette. Therefore, the system is not sufficient for the purpose of monitoring the conditions during the cell culture. Therefore, it is requested to develop an apparatus which can continuously monitor the conditions of cells under culture while preventing contamination from occurring and maintaining a clean condition.

### SUMMARY

According to the invention, there is provided a cell incubator comprising: a culture cell accommodating section which accommodates cells to be cultured and a culture solution; and an RFID tag which is attached to the culture cell accommodating section and which is configured to transmit predetermined information to a communicating apparatus in response to reception of a signal that is wirelessly transmitted from the communicating apparatus.

The cell incubator may further include a sensor which is configured to directly or indirectly measure at least one of a temperature of the culture solution, a carbon dioxide concentration, an oxygen concentration, an oxygen partial pressure, a glucose concentration, a lactate concentration, and an ammonia concentration. The RFID tag may be configured to transmit the predetermined information that includes a result of measurement by the sensor to the communicating apparatus.

The sensor may be incorporated in the RFID tag.

The RFID tag may include: a storage section which stores the predetermined information; and an information transmitting section which, in response to reception of the signal that is transmitted from the communicating apparatus, is configured to read out the predetermined information stored in the storage section and transmit the predetermined information to the communicating apparatus.

The storage section may store the predetermined information that includes at least an identification code and information related to a history of passage of the cells.

Contents of the predetermined information stored in the storage section may be rewritable in accordance with contents of the signal that is transmitted from the communicating apparatus.

The RFID tag may include a driving current producing section which is configured to convert a part of the signal that is transmitted from the communicating apparatus to a direct current, and the information transmitting section may be configured to transmit the predetermined information to the communicating apparatus, using the direct current which is converted by the driving current producing section as a driving current.

The cell incubator may further include a measuring unit which includes a light emitting section and a light receiving section, the measuring unit in which the light emitting section transmits light through the culture solution and the light receiving section receives the transmitted light, thereby measuring a condition of the culture solution, the measuring unit which is accommodated in a measuring unit accommodating section disposed integrally with the culture cell accommodating section, the measuring unit which is configured to transmit a result of measurement to the communicating apparatus.

The RFID tag may be configured to transmit the predetermined information to the communicating apparatus through the measuring unit.

The cell incubator may further include a measuring unit which includes a light emitting section and a light receiving section, the measuring unit in which the light emitting section transmits light through the culture solution and the light receiving section receives the transmitted light, thereby measuring a condition of the culture solution, the measuring unit which is accommodated in a measuring unit accommodating section disposed integrally with the culture cell accommodating section. The RFID tag may be configured to transmit the predetermined information that includes a result of measurement by the measuring unit to the communicating apparatus.

The measuring unit may be configured to measure a pH of the culture solution.

According to the invention, there is provided a cell incubator comprising: a culture cell accommodating section which accommodates cells to be cultured and a culture solution; and a measuring unit accommodating section which accommodates a measuring unit that includes a light emitting section and a light receiving section, the measuring unit in which the light emitting section transmits light through the culture solution and the light receiving section receives the transmitted light, thereby measuring a condition of the culture solution, wherein a part of the culture cell accommodating section is located between the light emitting section and light receiving section.

The part of the culture cell accommodating section may be projected between the light emitting section and light receiving section.

The cell incubator may further include an RFID tag which is attached to the culture cell accommodating section and which is configured to transmit predetermined information to a communicating apparatus in response to reception of a signal that is wirelessly transmitted from the communicating apparatus.

According to the invention, there is provided an incubation condition monitoring system comprising: the cell incubator; and the communicating apparatus which is configured to receive the predetermined information from the RFID tag by transmitting the signal to the RFID tag.

The incubation condition monitoring system may further include an incubator which accommodates a plurality of the cell incubator. The communicating apparatus may be disposed in the incubator.

The communicating apparatus may produce an alarm signal in accordance with a result of measurement by a measuring unit or a sensor, the measuring unit which includes a light emitting section and a light receiving section, the measuring unit in which the light emitting section transmits light through the culture solution and the light receiving section receives the transmitted light, thereby measuring a condition of the culture solution, the sensor which is configured to directly or indirectly measure at least one of a temperature of the culture solution, a carbon dioxide concentration, an oxygen concentration, an oxygen partial pressure, a glucose concentration, a lactate concentration, and an ammonia concentration.

### BRIEF DESCRIPTION OF THE DRAWINGS

Fig. 1 is a schematic diagram of an incubation condition monitoring system of an embodiment of the invention.
Fig. 2 is a perspective view of a cell incubator.
Fig. 3 is a plan view of an RFID tag.
Fig. 4 is a block diagram showing a configuration example of the RFID tag.
Fig. 5 is a block diagram showing another configuration example of the RFID tag which can acquire results of measurement by a measuring unit.

### DETAILED DESCRIPTION OF EMBODIMENTS

Hereinafter, the invention will be described by way of an embodiment thereof. However, the following embodiment is not intended to limit the invention as defined in the appended claims, and all combinations features described in the embodiment are not always essential to solving means of the invention.

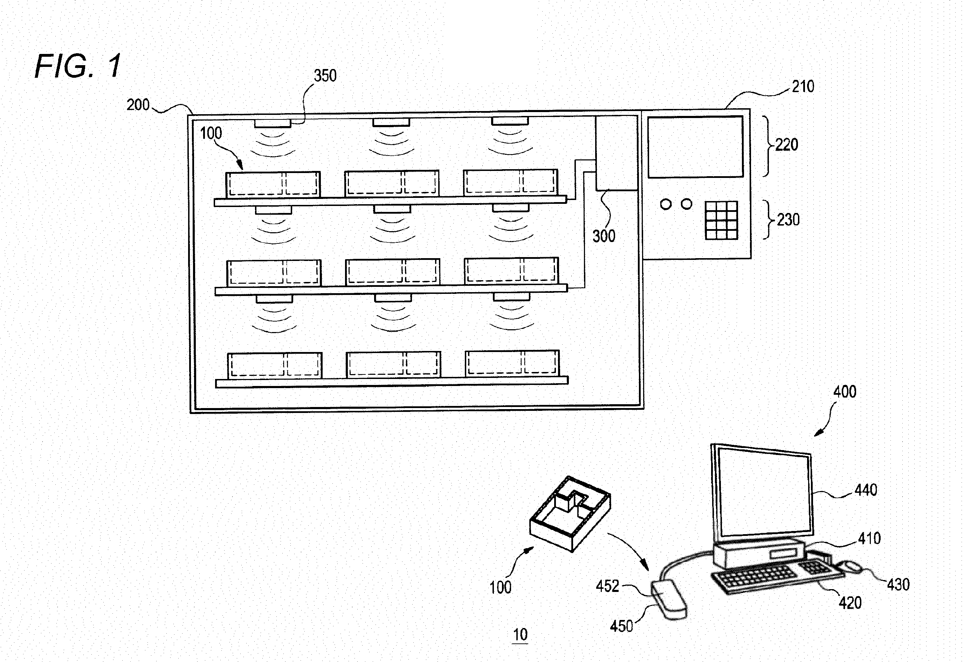

Fig. 1 is a schematic diagram of an incubation condition monitoring system 10 of the embodiment of the invention. The incubation condition monitoring system 10 includes: an incubator 200 which accommodates a plurality of cell incubators 100; an incubation condition monitoring apparatus 300 which is disposed in the incubator 200; and a data managing apparatus 400 which can read and write information stored in RFID tags 130 (described later) that are attached to the cell incubators 100, respectively, and can monitor the cell incubators 100 in which the kind of accommodated cells, the history of passage, and the like are different.

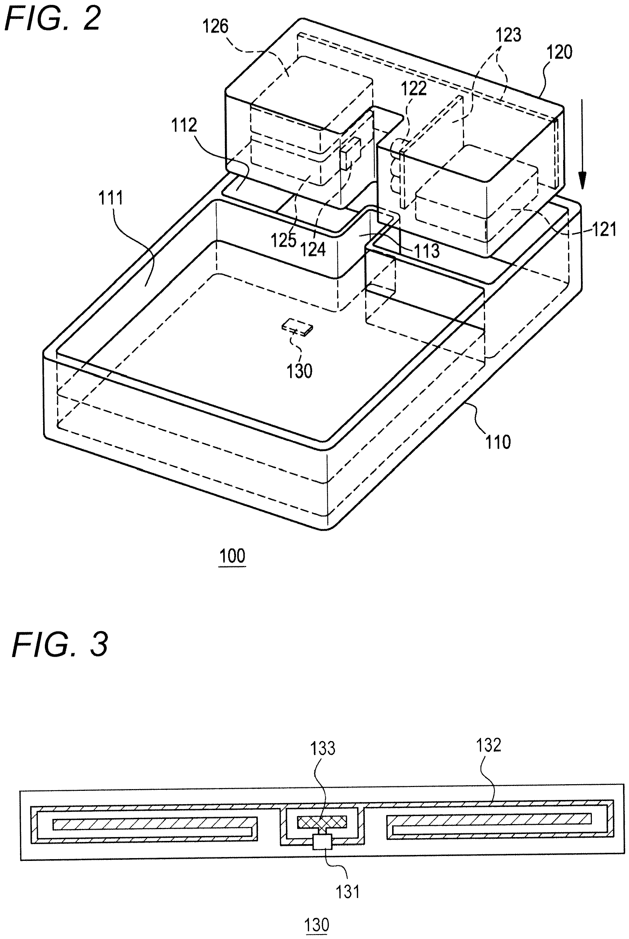

Fig. 2 is a perspective view showing one of the cell incubators 100. The cell incubator 100 includes an incubator body unit 110 configured by a culture cell accommodating section 111 and a measurement unit accommodating section 112; a pH measuring unit 120; and the RFID tag 130 which is attached to a position corresponding to the rear side of the culture cell accommodating section 111, in the lower surface of the incubator body unit 110. The cell incubator 100 in the example includes the pH measuring unit 120 which is an example of the measuring unit, and which can measure the pH of a culture solution. Alternatively, the cell incubator 100 may include a measuring unit which can measure an item other than the pH, in place of or together with the pH measuring unit 120.

In the incubator body unit 110, the culture cell accommodating section 111 accommodates cells which are to be cultivated in the cell incubator 100, and the culture solution. The measurement unit accommodating section 112 is disposed adjacent to and integrally with the culture cell accommodating section 111, and accommodates the pH measuring unit 120.

The pH measuring unit 120 includes: a battery 121 which supplies a driving power to various sections of the pH measuring unit 120; a plurality (in this example, three) of LEDs 122 which respectively emit light at different wavelengths; a circuit board 123 which performs functions such as a lighting control of the LEDs 122; a light receiving element 124 which receives light emitted from the LEDs 122; a signal processing circuit section 125 which calculates the pH of the culture solution from the reception light output supplied from the light receiving element 124 ; and a transmitting section 126 which transmits the result of the measurement of the pH of the culture solution. The culture solution in the culture cell accommodating section 111 contains a pH indicator. One of the three LEDs 122 is an offset LED which emits light at a wavelength that shows a very little absorption by the indicator.

One of the other two LEDs 122 emits light at a wavelength that shows a more conspicuous absorption by the indicator as the pH of the culture solution moves further toward the acidic side of the median value of a predetermined management width, and the other LED emits light at a wavelength that shows a more conspicuous absorption by the indicator as the pH of the culture solution moves further toward the alkaline side of the median value. As the indicator, for example, phenol red is preferably used. In the case where phenol red is used as the indicator, among the three LEDs 122, for example, the offset LED emits light in a 700 nm band, and the remaining two LEDs emit light in 558 nm and 430 nm bands.

When the pH of the culture solution is to be observed by the pH measuring unit 120, the offset LED is first caused to emit light, and a calibration coefficient of the absorption by the culture solution is obtained. Here, the temperature of the culture solution may be varied, and the calibration coefficient may be obtained as a function of the temperature. Next, the remaining two LEDs are caused to alternately emit light at predetermined intervals, and the light receiving element 124 receives light which is transmitted through the culture solution in a projection 113 that is projected toward the measurement unit accommodating section 112 in the culture cell accommodating section 111. Preferably, the projection 113 has a shape which is located between the light receiving element 124 and the LEDs 122 of the pH measuring unit 120 so that the optical axes between the LEDs 122 of the pH measuring unit 120 and the light receiving element intersect with a part (the projection 113) of the culture cell accommodating section 111. According to the configuration, the pH measuring unit 120 can continuously monitor the situation of the culture cell accommodating section 111 in which cells are cultured.

Then, the signal processing circuit section 125 calculates the absorbances at the light emission wavelengths of the LEDs, based on the reception light output from the light receiving element 124, and further calculates the pH of the culture solution based on previously obtained relationships in the indicator between the pH and the absorbance, and the calibration coefficient. Then, the value of the calculated pH is correlated with information indicative of measurement conditions such as the measurement time (for example, the light emitting timings of the LEDs), and thereafter transmitted to the incubation condition monitoring apparatus 300 by the transmitting section 126. The method of communication between the transmitting section 126 and the incubation condition monitoring apparatus 300 is selected from various short-range wireless communication methods such as infrared (IrDA) communication, Bluetooth, and wireless LAN. However, the method is not particularly limited. The transmitting section 126 may transmit also results of measurement performed by a sensor incorporated in the RFID tag.

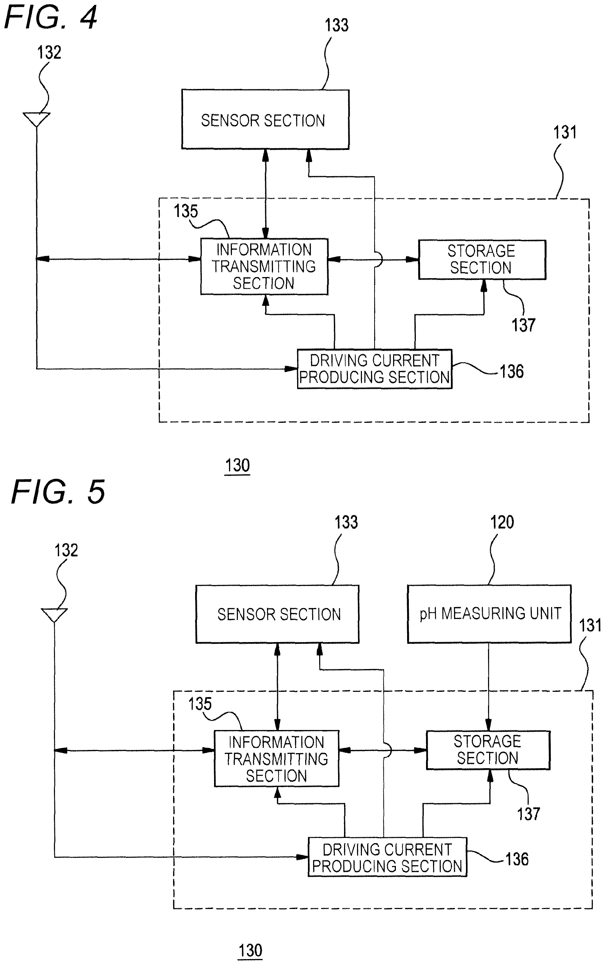

Fig. 3 is a plan view of the RFID tag 130, and Fig. 4 is a block diagram showing a configuration example of the RFID tag 130. As described above, the RFID tag 130 is attached to the position corresponding to the rear side of the culture cell accommodating section 111, in the lower surface of the incubator body unit 110, and, as shown in Fig. 3, includes an IC chip 131, a transmission/reception antenna 132, and a sensor section 133.

The sensor section 133 receives a supply of a driving current from a driving current producing section 136 which will be described later, measures the condition of the culture solution in the culture cell accommodating section 111, and outputs the condition to an information transmitting section 135. In this example, the sensor section 133 includes a temperature sensor which indirectly measures the temperature of the culture solution in the culture cell accommodating section 111. The sensor section 133 may further include a carbon dioxide sensor which measures the CO₂ concentration in the periphery of the culture cell accommodating section 111, or may be a sensor which can measure the oxygen concentration, the oxygen partial pressure, the glucose concentration, the lactate concentration, the ammonia concentration, and the like. The sensor section 133 is requested to be placed at a position where the objective measurement item can be normally measured, and preferably disposed on the wall surface, the rear side, or the like in accordance with the measurement item.

As shown in Fig. 4, the IC chip 131 has the information transmitting section 135, the driving current producing section 136, and a storage section 137. In response to reception of the signal through the transmission/reception antenna 132 from a communicating apparatuses 350 which are disposed in a plural number in the incubator 200, and which wirelessly transmit a signal, the information transmitting section 135 transmits predetermined information to the communicating apparatuses 350. When a signal instructing transmission of results of measurement by the sensor section 133 is transmitted from the communicating apparatuses 350, more specifically, the information transmitting section 135 obtains the results of measurement by the sensor section 133, and transmits the measurement results to the communicating apparatuses 350 through the transmission/reception antenna 132. The information transmitting section 135 may transmit the results of measurement by the sensor section 133 to the transmitting section 126 of the pH measuring unit 120, and the transmitting section 126 may transmit the information transmitted from the information transmitting section 135 to the communicating apparatuses 350, either singly or together with results of measurement by the pH measuring unit 120. Namely, the RFID tag 130 may transmit information stored in itself to the communicating apparatuses 350 through the transmitting section 126 of the pH measuring unit 120. The transmitting section 126 may perform transmission to the communicating apparatuses 350 in a wired manner.

When a signal instructing transmission of information stored in the storage section 137 is transmitted from the communicating apparatuses 350, for example, the information transmitting section 135 acquires information stored in the storage section 137, and transmits the information to the communicating apparatuses 350 through the transmission/reception antenna 132. When a signal instructing rewrite of information stored in the storage section 137 is transmitted from the communicating apparatuses 350, the information transmitting section 135 rewrites information stored in the storage section 137 in accordance with the contents of the signal.

The storage section 137 is a memory which is rewritable by electrical means, and stores information such as the ID (identification code) unique to the cell incubator 100, information related to cells in the culture cell accommodating section 111 (for example, information of the patient from whom cells are taken out, and the history of passage of cells), and information indicative of the stage in the culturing step where cells in the cell incubator 100 exist. The stored contents are adequately rewritten by the information transmitting section 135.

As the storage section 137, for example, an EEPROM (ELECTRICALLY Erasable Programmable Read Only Memory) is preferably used. However, the storage section is not limited to this, and may be an FRAM (Ferroelectric Random Access Memory) which is a ferroelectric memory, or the like may be used.

In the case where information stored in the storage section 137 is set not to be rewritable, a mask ROM (Read Only Memory) or the like is preferably used as the storage section 137. The storage section 137 may store the results of measurement performed by the sensor section 133.

The driving current producing section 136 converts a part of a signal wave transmitted from the communicating apparatuses 350 to a direct current, and supplies the direct current to the sensor section 133, the information transmitting section 135, and the storage section 137 as a driving current for the sections. The RFID tag 130 may include a small battery in place of the driving current producing section 136 in the example.

The incubation condition monitoring apparatus 300 outputs the signal which is to be transmitted to the RFID tag 130 of the cell incubator 100, to the corresponding communicating apparatus 350. Moreover, the incubation condition monitoring apparatus 300 acquires the signal which is received from the RFID tag 130 by the communicating apparatus 350. The contents of the signal which is transmitted from the RFID tag 130 of the cell incubator 100 are displayed on a display panel 220 of a controlling apparatus 210 which is disposed outside the incubator 200. The contents of the signal which is to be transmitted from the incubation condition monitoring apparatus 300 to the RFID tag 130 of the cell incubator 100 through the communicating apparatus 350 can be set or changed through operation buttons 230 of the controlling apparatus 210 or the like.

If it is determined that there is an abnormality in the signal received from the RFID tag 130 or the pH measuring unit 120, the incubation condition monitoring apparatus 300 produces an alarm signal, and the apparatus itself may output the alarm, or the data managing apparatus 400 which will be described later may output the alarm. Moreover, the alarm may be transmitted to a portable telephone of the person in charge. The configuration where the incubation condition monitoring apparatus 300 has such a function of outputting an alarm enables the person in charge to, in the case where results of measurement by the pH measuring unit 120 or the sensor section 133 contain an abnormal value, for example, easily know the condition.

The data managing apparatus 400 is configured by an apparatus body unit 410, a keyboard 420, a mouse 430, a display 440, and a reader/writer 450. When the cell incubator 100 which is taken out from the incubator 200 is placed in front of the reader/writer 450 so that the surface onto which the RFID tag 130 is attached is opposed to a non-contact communication surface 452 of the reader/writer 450, whereby information such as that related to cells which are cultured by the cell incubator 100, and the above-described various measurement results can be read from the RFID tag 130. At this time, the reader/writer 450 can newly write information into the RFID tag 130.

The contents of information to be written into the RFID tag 130 can be set or changed by using an inputting apparatus such as the keyboard 420 or the mouse 430. The contents of the information which is obtained from the RFID tag 130 by the reader/writer 450 are displayed on the display 440. In the case where the alarm signal has been already produced, the alarm may be displayed.

As described above, according to the incubation condition monitoring system 10 of the embodiment, even in the case where a plurality of cell incubators 100 are stored in the incubator 200, for example, information such as the kind of cells in each of the cell incubators 100, and the history of passage can be easily obtained through the communicating apparatuses 350 in the incubator \200. While the cell culture is performed in the plurality of cell incubators 100, therefore, it is possible to easily manage the culture environment and the history. The application of the cell incubator and system of the invention is not limited to monitoring in an incubator, and the cell incubator and the system may be applied to any other step required in the cell culture, such as the dispensing step or the seeding step.

The RFID tag 130 attached to the cell incubator 100 which is used in the incubation condition monitoring system 10 of the example incorporates the sensor which measures the temperature of the culture solution and the like, and therefore the whole cell incubator 100 can be further miniaturized as compared with the case where a temperature sensor and the like are separately disposed. The information stored in the storage section 137 of the RFID tag 130 can be rewritten as needed. In the case where, for example, passage is performed or the kind of cells to be cultured is changed, therefore, the information stored in the storage section 137 such as the kind of cells, and the history of passage can be easily updated.

In the example, the pH measuring unit 120 transmits the results of measurement of the pH of the culture solution by means of the transmitting section 126 which is provided to the unit itself. As shown in a configuration example of the block diagram of Fig. 5, for example, the results of pH measurement by the pH measuring unit 120 may be stored in the storage section 137 of the RFID tag 130. In the case of the example, the results of measurement by the pH measuring unit 120 are read out together with other information from the storage section 137 by the information transmitting section 135, and transmitted to the communicating apparatuses 350. In the example, the transmitting section 126 is required to be disposed in the pH measuring unit 120, and hence the pH measuring unit 120 can be further miniaturized.

Although the invention has been described using the embodiment, the technical scope of the invention is not restricted to the scope of the description of the embodiment. It is obvious to those skilled in the art that various changes or improvements can be made on the embodiment.

According to an aspect of the invention, even in the case where a plurality of cell incubators are stored in an incubator, for example, information such as the kind of cells in each container and the history of passage can be obtained easily and cleanly without contamination, from the RFID tag of the cell incubator by using the communicating apparatus which is a reading apparatus or the like.

According to an aspect of the invention, in the case where the cell incubator is stored in an incubator, for example, various measurement values such as the temperature of the culture solution in the cell incubator and the concentration of carbon dioxide in the periphery of the cell incubator can be obtained without taking out the cell incubator from the incubator.

According to an aspect of the invention, the sensor and the configuration for transmitting a result of measurement by the sensor are integrated with each other by the RFID tag, and therefore the RFID tag attached to the culture cell accommodating section can be further miniaturized.

According to an aspect of the invention, information of each cell incubator such as an identification code of the cell incubator, the kind of cells in the cell incubator, the history of passage, and the result of measurement by the sensor is stored in the storage section of the RFID tag, and the information can be easily obtained through the information transmitting section. Also, the workability of the monitoring person is improved.

According to an aspect of the invention, in the case where the communicating apparatus is provided with a writing function, and passage is newly performed or the kind of cells to be cultured is changed, for example, information which is stored in the storage section, such as the kind of the cells and the history of passage can be easily updated. Moreover, the time and date of an exchange of the culture medium, and the situations which have occurred can be correctly known, and furthermore prediction of the timing of the next exchange of the culture medium, and analysis of the tendency can be performed based on information which is accumulated in the storage section.

According to an aspect of the invention, it is not necessary to dispose a battery in the RFID tag, or to supply an electric power from an external power supply to the RFID tag, thereby enabling continuous monitoring to be performed for a long term.

According to an aspect of the invention, also the result of the measurement performed on the culture solution by the measuring unit, and the information stored in the RFID tag can be remotely obtained without taking out the cell incubator. Moreover, the measuring unit is accommodated in the measuring unit accommodating section which is disposed integrally with the culture cell accommodating section. While enabling the condition of the culture solution to be measured, therefore, the structure of the whole cell incubator is prevented from being complicated and enlarged.

According to an aspect of the invention, the result of the measurement performed on the culture solution by the measuring unit can be remotely obtained without taking out the cell incubator.

According to an aspect of the invention, the result of the measurement of the pH of the culture solution performed by the measuring unit can be remotely obtained without taking out the cell incubator, and the condition of the culture medium can be known.

According to an aspect of the invention, even in the case where a plurality of cell incubators are stored in the incubator, for example, information such as the kind of cells in each container and the history of passage can be easily obtained and managed by using the communicating apparatus which is a reading apparatus or the like. This can contribute to reduction of human errors such as misidentification of patients from whom cells are taken out, and misunderstanding of cultured portions.

According to an aspect of the invention, in the case where an abnormal value is contained in the measurement result, for example, the administrator can easily know such a situation by means of an alarm, and contamination can be prevented from expanding.

The above description of the invention does not list all the necessary features of the invention, but sub-combinations of a group of these features can also constitute the invention.

## Claims

1. A cell incubator comprising:
a culture cell accommodating section which accommodates cells to be cultured and a culture solution; and
an RFID tag which is attached to the culture cell accommodating section and which is configured to transmit predetermined information to a communicating apparatus in response to reception of a signal that is wirelessly transmitted from the communicating apparatus.

2. The cell incubator according to claim 1, further comprising a sensor which is configured to directly or indirectly measure at least one of a temperature of the culture solution, a carbon dioxide concentration, an oxygen concentration, an oxygen partial pressure, a glucose concentration, a lactate concentration, and an ammonia concentration,
wherein the RFID tag is configured to transmit the predetermined information that includes a result of measurement by the sensor to the communicating apparatus.

3. The cell incubator according to claim 2, wherein the sensor is incorporated in the RFID tag.

4. The cell incubator according to claim 1, wherein the RFID tag includes:
a storage section which stores the predetermined information; and
an information transmitting section which, in response to reception of the signal that is transmitted from the communicating apparatus, is configured to read out the predetermined information stored in the storage section and transmit the predetermined information to the communicating apparatus.

5. The cell incubator according to claim 4, wherein the storage section stores the predetermined information that includes at least an identification code and information related to a history of passage of the cells.

6. The cell incubator according to claim 4, wherein contents of the predetermined information stored in the storage section are rewritable in accordance with contents of the signal that is transmitted from the communicating apparatus.

7. The cell incubator according to claim 4, wherein
the RFID tag includes a driving current producing section which is configured to convert a part of the signal that is transmitted from the communicating apparatus to a direct current, and
the information transmitting section is configured to transmit the predetermined information to the communicating apparatus, using the direct current which is converted by the driving current producing section as a driving current.

8. The cell incubator according to claim 1, further comprising a measuring unit which includes a light emitting section and a light receiving section, the measuring unit in which the light emitting section transmits light through the culture solution and the light receiving section receives the transmitted light, thereby measuring a condition of the culture solution, the measuring unit which is accommodated in a measuring unit accommodating section disposed integrally with the culture cell accommodating section, the measuring unit which is configured to transmit a result of measurement to the communicating apparatus.

9. The cell incubator according to claim 8, wherein the RFID tag is configured to transmit the predetermined information to the communicating apparatus through the measuring unit.

10. The cell incubator according to claim 8, wherein the measuring unit is configured to measure a pH of the culture solution.

11. The cell incubator according to claim 1, further comprising a measuring unit which includes a light emitting section and a light receiving section, the measuring unit in which the light emitting section transmits light through the culture solution and the light receiving section receives the transmitted light, thereby measuring a condition of the culture solution, the measuring unit which is accommodated in a measuring unit accommodating section disposed integrally with the culture cell accommodating section,
wherein the RFID tag is configured to transmit the predetermined information that includes a result of measurement by the measuring unit to the communicating apparatus.

12. The cell incubator according to claim 11, wherein the measuring unit is configured to measure a pH of the culture solution.

13. A cell incubator comprising:
a culture cell accommodating section which accommodates cells to be cultured and a culture solution; and
a measuring unit accommodating section which accommodates a measuring unit that includes a light emitting section and a light receiving section, the measuring unit in which the light emitting section transmits light through the culture solution and the light receiving section receives the transmitted light, thereby measuring a condition of the culture solution, wherein
a part of the culture cell accommodating section is located between the light emitting section and light receiving section.

14. The cell incubator according to claim 13, wherein
the part of the culture cell accommodating section is projected between the light emitting section and light receiving section.

15. The cell incubator according to claim 13, further comprising an RFID tag which is attached to the culture cell accommodating section and which is configured to transmit predetermined information to a communicating apparatus in response to reception of a signal that is wirelessly transmitted from the communicating apparatus.

16. An incubation condition monitoring system comprising:
the cell incubator according to claim 1; and
the communicating apparatus which is configured to receive the predetermined information from the RFID tag by transmitting the signal to the RFID tag.

17. The incubation condition monitoring system according to claim 16, further comprising an incubator which accommodates a plurality of the cell incubator,
wherein the communicating apparatus is disposed in the incubator.

18. The incubation condition monitoring system according to claim 16, wherein the communicating apparatus produces an alarm signal in accordance with a result of measurement by a measuring unit or a sensor, the measuring unit which includes a light emitting section and a light receiving section, the measuring unit in which the light emitting section transmits light through the culture solution and the light receiving section receives the transmitted light, thereby measuring a condition of the culture solution, the sensor which is configured to directly or indirectly measure at least one of a temperature of the culture solution, a carbon dioxide concentration, an oxygen concentration, an oxygen partial pressure, a glucose concentration, a lactate concentration, and an ammonia concentration.
